# FASCICULE DE BREVET EUROPEEN

(11) **EP 0 633 928 B1**
(45) Date de publication et mention de la délivrance du brevet: **19.11.2003**
(21) Numéro de dépôt: 93907916.6
(22) Date de dépôt: 31.03.1993
(51) Int. Cl.: C12N 1/14, C12N 15/01, C12N 15/09, C07D 498/06, C07K 7/54

(54) **MICROORGANISMES, PROCEDE DE PREPARATION ET UTILISATION**
MIKROORGANISMEN, IHRE HERSTELLUNG UND VERWENDUNG
MICRO-ORGANISMS, PREPARATION METHOD THEREFOR AND USES THEREOF

(30) Priorité: 01.04.1992 FR 9203939
(43) Date de publication de la demande: 18.01.1995
(73) Titulaire: Aventis Pharma S.A., 92160 Antony (FR)
(72) Inventeur: BARRERE, Geneviève, F-75005 Paris (FR); JUMEL, Catherine, F-31750 Escalquens (FR); LACROIX, Patricia, F-94360 Bry-sur-Marne (FR); LEHMANN, Corinne, F-91700 Ste.-Geneviève-des-Bois (FR); SABATIER, Alain, F-75013 Paris (FR)
(74) Mandataire: Becker, Philippe
(86) Numéro de dépôt international: FR9300324
(87) Numéro de publication internationale: WO93020182

(56) Documents cités:
- EP-A- 0 248 703
- US-A- 4 355 112
- BIOTECHNOLOGY ABSTRACTS, Derwent Publications Ltd., London, GB, abstract no. 84-07062, "Selective production of antibiotic neoviridogrisein II - using propylhydroxylase-deficient Streptomyces sp. strain obtained by chemical or radiation mutagenesis", & JP - A - 59059198 (SANRAKU-OCEAN) 04.04.1984, abrégé
- FOLIA MICROBIOLOGICA, vol. 35, no. 6, 1990, page 494, Prague, TCHECOSLOVAQUIE, M. BLUMAUEROVA et al.: "Physiological and genetic aspects of virginiamycin production",abrégé
- DATABASE WPI, week 6800, Derwent Publications Ltd., London, GB, AN 66-10866F, & FR - A - 1349946 (KANEGAFUCHI CHEM IND CO LTD), voir abrégé
- THE JOURNAL OF ANTIBIOTICS SERIES A, vol. XIII, no. 1, janvier 1960, pages 62-69, Tokyo, JP, K. WATANABE: "Studies on mikamycin. V In vitro synergistic action and differential assay of mikamycin components", document entier (cité dans la demande)
- BIOTECHNOLOGY OF INDUSTRIAL ANTIBIOTICS, vol. 22, 1984, pages 695-720, A.M. BIOT: "Virginiamycin: properties, biosynthesis, and fermentation", document entier (cité dans la demande)

## Description

La présente invention concerne de nouveaux microorganismes, un procédé pour leur préparation ainsi que leur utilisation. Plus particulièrement, l'invention concerne des microorganismes producteurs d'antibiotiques.

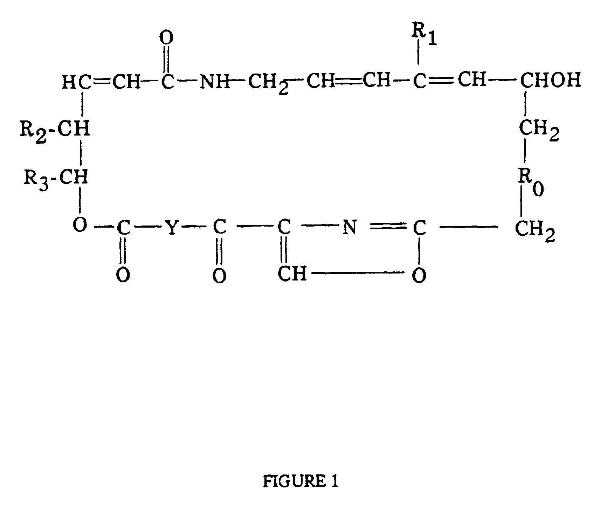

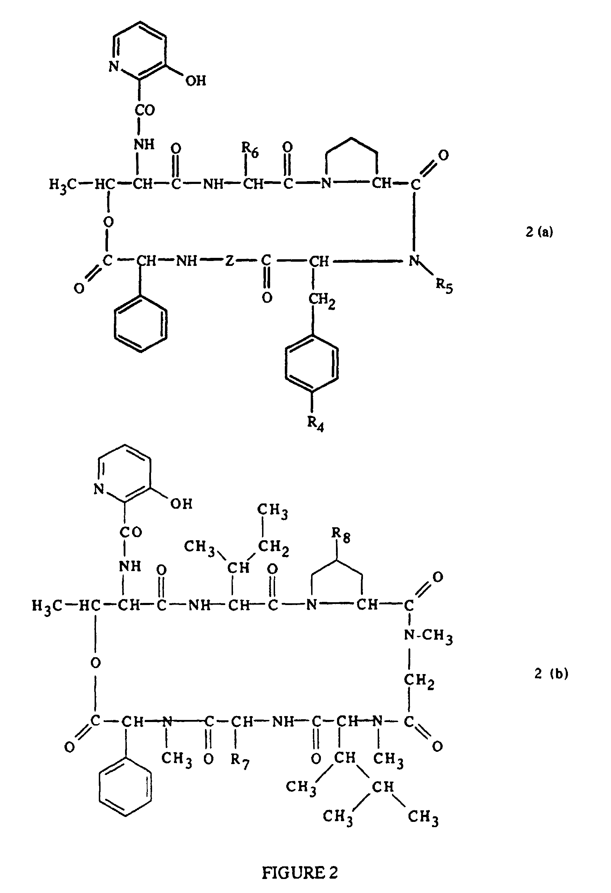

Parmi les nombreux antibiotiques décrits dans l'art antérieur, certains présentent la particularité d'être constitués de plusieurs composantes actives agissant ensemble de manière synergique. C'est le cas notamment des antibiotiques de la famille des streptogramines, qui sont composés d'une macrolactone (composante du groupe A) et d'un depsipeptide (composante du groupe B). L'activité antimicrobienne et le mode d'action de ces antibiotiques ont fait l'objet d'études, notamment par Tanaka (Antibiotics, vol III p.487, Springer, Berlin (1975)) et Vasquez (Antibiotics, vol III p.521, Springer, Berlin (1975)). La structure générale des molécules des groupes A et B est représentée sur les figures 1 et 2.

Parmi les antibiotiques connus de la famille des streptogramines, on peut citer plus particulièrement :
- les pristinamycines,
- les mikamycines,
- les virginiamycines,
- les vernamycines,
- les ostréogrycines,
- les synergistines,
- les plauracines,
- les étamycines,
- les streptogramines,
- les viridogriséines,
- les griséoviridines, ou,
- les néoviridogriséines.

La forme active de chacun de ces antibiotiques est constituée de l'association synergique de molécules appartenant au groupe A telles que représentées sur la figure 1 et de molécules appartenant au groupe B telles que représentées sur la figure 2, ou de molécules apparentées.

Par molécules apparentées, on entend au sens de la présente invention, des molécules présentant le squelette général de celles représentées sur les figures 1 et 2, mais pouvant se distinguer de celles-ci par des substitutions ou d'autres variations secondaires, et possédant une activité de même nature.

Les antibiotiques de la famille des streptogramines sont produits par une grande variété de microorganismes, et en particulier, par les bactéries du genre Actinomycète et par certains champignons. Ces microorganismes sont caractérisés par le fait qu'ils synthétisent de manière simultanée les deux composantes A et B.

Le tableau 1 mentionne les principaux microorganismes producteurs ainsi que les antibiotiques correspondants.

Des études ont été réalisées dans le but d'augmenter les niveaux de production de ces microorganismes. On peut citer notamment les travaux de Biot (Biotechnology of Industrial Antibiotics, vo1.22 (1984) p.695) ou de Prikrylova et al (Biotechnology and Bioindustry/2 (1988) 20) portant sur l'amélioration des conditions de culture et sur l'effet d'agents mutagéniques sur les niveaux de production de virginiamycine par S.virginiae. Comme l'indiquent les auteurs, les mutants surproducteurs obtenus produisent toujours simultanément les composantes AetB.

Cependant, le fait que les microorganismes producteurs de streptogramines disponibles dans l'art antérieur synthétisent simultanément, dans le même milieu de fermentation, les deux composantes synergiques A et B de l'antibiotique constitue dans certains cas un inconvénient important.

En effet, pour valoriser ces antibiotiques et pouvoir les utiliser comme agents pharmaceutiques, il est préférable de pouvoir séparer et purifier les composantes A et B des streptogramines. Ceci est également indispensable pour pouvoir réaliser des études chimiques sur les Streptogramines, en particulier dans le but de préparer des dérivés hémisynthétiques, comme par exemple ceux décrits dans les brevets FR 2,549,063 ; FR 2,549,065; EP 191662 ou EP 248 703.

Cependant, l'accessibilité à ces différentes composantes est difficile, en raison notamment de leur production simultanée et de la similitude de leurs propriétés physicochimiques. De plus, ces microorganismes synthétisent généralement plusieurs molécules différentes de chaque composante, conduisant dans le moût de fermentation à un mélange de nombreux composés, dans des proportions très variables. On connaît en effet aujourd'hui de nombreuses molécules appartenant aux groupes A et B des streptogramines, possédant des activités biologiques très différentes, qui sont produites simultanément par les microorganismes. Ainsi, on connaît dans l'art antérieur les molécules suivantes appartenant au groupe A : les pristinamycines PIIA et PIIB, les virginiamycines M1 et M2, la mikamycine A ou les ostréogrycines A et G. De même, on connaît dans l'art antérieur les molécules suivantes appartenant au groupe B : les pristinamycines PIA, PIB, et PIC ; les virginiamycines S1, S2, S3, S4 et S5; la mikamycine B; les vernamycines Bα, Bβ, Bγ et Bδ; les ostréogrycine B, B1, B2 et B3 ou les néoviridogriséines I, II, III et IV.

Pour ces raisons, il est difficile d'obtenir de manière satisfaisante au niveau industriel des mélanges synergiques actifs et pharmaceutiquement acceptables de streptogramines. Il est également difficile de réaliser des études chimiques sur les streptogramines (relation structure-fonction, mise au point de formes hydrosolubles, etc). La présente invention permet de remédier aux inconvénients de l'art antérieur dans ce domaine.

La Demanderesse a maintenant montré qu'il est possible d'obtenir des microorganismes produisant séparément les composantes A et B des streptogramines de façon stable. La Demanderesse a donc mis au point, et ceci constitue un objet de la présente invention, des microorganismes capables de produire sélectivement les composantes A et B des streptogramines. L'invention concerne aussi bien les microorganismes capables de produire sélectivement les molécules du groupe A des streptogramines ou les molécules du groupe B des streptogramines que les microorganismes capables de produire sélectivement une molécule spécifique de l'un de ces groupes.

L'invention permet ainsi la production séparée, en quantités importantes, de streptogramine A ou B.

L'invention permet également l'utilisation de procédés de purification des streptogramines plus simples et donc plus performants et plus économiques, puisqu'il n'y a plus ou moins d'interactions entre les différentes composantes au cours de l'extraction.

L'invention permet aussi de faire varier les quantités respectives des composantes A et B dans un mélange, et donc de réaliser des compositions antibiotiques optimales, au niveau de la pureté comme de l'efficacité synergique.

L'invention permet encore d'obtenir des produits en quantité et en pureté suffisantes pour réaliser des études chimiques afin d'améliorer encore les propriétés des streptogramines.

L'invention fournit ainsi un système de production de streptogramines permettant une exploitation industrielle de ces antibiotiques beaucoup plus performante.

Plus particulièrement, l'invention concerne les microorganismes capables de produire sélectivement les composantes A et B des streptogramines choisies parmi le groupe comprenant la pristinamycine, la virginiamycine, la mikamycine, l'ostréogrycine, la synergistine, la viridogriséine, la vemamycine, la plauracine, l'étamycine, la griséoviridine, la néoviridogriséine et la streptogramine.

Dans un mode préféré, l'invention concerne les microorganismes capables de produire sélectivement les composantes A des streptogramines correspondant à la formule générale représentée sur la figure 1 dans laquelle :
- Y représente une D-proline, une 4,5-déhydroproline, une D-alanine ou une D-cystéine,
- R0 représente un groupement C=O ou CHOH,
- R1 est un atome d'hydrogène ou un groupement méthyl,
- R2 est un atome d'hydrogène ou un groupement méthyl, et,
- R3 est un groupement méthyl ou isopropyl.

Dans un autre mode préféré, l'invention concerne les microorganismes capables de produire sélectivement les composantes B des streptogramines correspondant à la formule générale représentée sur la figure 2(a) dans laquelle :
- Z représente une L-proline, un acide aspartique L, un acide pipécolique, un acide 4-oxopipécolique, un acide 4-hydroxy-pipécolique L, ou un acide 5-hydroxy-4-oxopipécolique L,
- R4 est un atome d'hydrogène ou une amine de formule NH(CH₃) ou N(CH₃)₂,
- R5 est un atome d'hydrogène ou un groupement méthyl, et,
- R6 est un groupement méthyl ou éthyl.

Toujours dans un mode préféré, l'invention concerne les microorganismes capables de produire sélectivement les composantes B des streptogramines correspondant à la formule générale représentée sur la figure 2(b) dans laquelle :
- R7 représente un atome d'hydrogène ou un groupe hydroxy, et
- R8 représente un groupement méthyl ou éthyl.

Plus préférentiellement, l'invention concerne les microorganismes capables de produire sélectivement, seules ou en mélange, les composantes A ou B des streptogramines choisies parmi le groupe comprenant la pristinamycine, la virginiamycine, l'ostréogrycine et la mikamycine.

A titre d'exemple, l'invention concerne les microorganismes capables de produire sélectivement les composantes A des streptogramines correspondant à la formule générale présentée sur la figure 1 dans laquelle R0 représente un groupement C=O, R1 représente un groupement méthyl, R2 représente un groupement méthyl, R3 représente un groupement isopropyl et Y représente une D-proline ou une 4,5-déhydroproline ; et les microorganismes capables de produire sélectivement les composantes B des streptogramines correspondant à la formule générale présentée sur la figure 2(a) dans laquelle :
- R4 est une amine de formule N(CH₃)₂, R5 est un groupement méthyl, R6 est un groupement éthyl et Z représente un acide 4-oxopipécolique ; ou
- R4 est une aminé de formule NHCH₃, R5 est un groupement méthyl, R6 est un groupement éthyl et Z représente un acide 4-oxopipécolique ; ou
- R4 est une amine de formule N(CH₃)₂, R5 est un groupement méthyl, R6 est un groupement méthyl et Z représente un acide 4-oxopipécolique ; ou
- R4 est une amine de formule N(CH₃)₂, R5 est un groupement méthyl, R6 est un groupement éthyl et Z représente un acide 5-hydroxy-4-oxopipécolique L ; ou
- R4 est un atome d'hydrogène, R5 est un groupement méthyl, R6 est un groupement éthyl et Z représente un acide 4-oxopipécolique ; ou
- R4 est un atome d'hydrogène, R5 est un groupement méthyl, R6 est un groupement méthyl et Z représente un acide 4-oxopipécolique ; ou
- R4 est un atome d'hydrogène, R5 est un groupement méthyl, R6 est un groupement éthyl et Z représente un acide 5-hydroxy-4-oxopipécolique L ; ou
- R4 est un atome d'hydrogène, R5 est un atome d'hydrogène, R6 est un groupement éthyl et Z représente un acide 4-hydroxy-pipécolique L.

Préférentiellement, les microorganismes de l'invention sont essentiellement des bactéries du genre Actinomycète ou des champignons.

Encore plus préférentiellement, les microorganismes de l'invention appartiennent au genre Streptomyces.

Les microorganismes de l'invention peuvent être cultivés dans des conditions standard de fermentation aérobie. Notamment, le milieu nutritif est généralement constitué d'une source de carbone, d'une source d'azote, et de sels minéraux. Comme source de carbone, on peut utiliser en particulier des sucres, huiles, acides organiques, dextrine, amidons, glycérol, etc. Comme source d'azote, on peut citer les acides aminés, farines végétales et extraits (malte, soja, graines de coton, tomates, maïs, etc), viscères, hydrolysats variés (caséine, levure, etc), et sous produits industriels tels que "distillers solubles". Comme source minérale, on peut utiliser des chlorures, nitrates, carbonates, sulfates et phosphates de sodium, potassium, ammonium ou calcium, ou des éléments traces tels que magnésium, fer, cuivre, zinc, manganèse ou cobalt.

Les microorganismes de la présente invention sont préférentiellement obtenus à partir de microogranismes producteurs non-sélectifs de streptogramines.

Par microorganismes non-sélectifs, on entend au sens de la présente invention les microorganismes qui synthétisent simultanément les composantes A et B des streptogramines. Tous les microorganismes cités dans le tableau 1 constituent des microorganismes non-sélectifs au sens de l'invention. La demanderesse a maintenant montré que des microorganismes producteurs sélectifs de streptogramines peuvent être obtenus à partir de microorganismes non-sélectifs producteurs de streptogramines. A cet effet, un procédé original a été développé impliquant une première étape facultative de mutagénèse, suivie d'une étape de sélection.

Un autre objet de la présente invention concerne donc un procédé de préparation des microorganismes définis ci-dessus, selon lequel on réalise les étapes suivantes :
- dans une première étape facultative, on effectue une mutagénèse sur un microorganisme producteur de streptogramines non-sélectif, et,
- dans une deuxième étape, on sélectionne les microorganismes sélectifs.

Comme indiqué plus haut, les microorganismes non-sélectifs sont généralement regroupés parmi les Actinomycètes et les champignons. Plus préférentiellement, les microorganismes de départ utilisables dans le procédé de l'invention sont des microorganismes producteurs non-sélectifs d'une streptogramine choisie parmi le groupe comprenant la pristinamycine, la virginiamycine, la mikamycine, l'ostréogrycine, la synergistine, la viridogriséine, la vernamycine, la plauracine, l'étamycine, la néoviridogriséine, la griséoviridine et la streptogramine. A titre d'exemple, les microorganismes cités dans le tableau 1 constituent des microorganismes non-sélectifs utilisables dans le procédé de l'invention. Encore plus préférentiellement, le procédé de l'invention est mis en oeuvre à partir de microorganismes choisis parmi Streptomyces alborectus, Streptomyces diastaticus, Streptomyces graminofaciens, Streptomyces griseus, Streptomyces loïdensis, Streptomyces mitakaensis, Streptomyces olivaceus, Streptomyces ostréogriseus, Streptomyces pristinaespiralis, Streptomyces virginiae, Streptomyces lavendulae, Streptomyces griséoviridus, Micromonospora sp., Actinomyces auranticolor, Actinomyces daghestanicus et Actinomyces azureus.

Encore plus préférentiellement, le procédé de l'invention est mis en oeuvre à partir de microorganismes choisis parmi Streptomyces alborectus, Streptomyces mitakaensis, Streptomyces pristinaespiralis, Streptomyces ostréogriséus et Streptomyces virginiae.

La première étape du procédé consiste à modifier le microorganisme non-sélectif, de manière à augmenter ces capacités globales de production d'antibiotique, et/ou pour qu'il ne synthétise qu'une seule des 2 composantes des streptogramines. Ceci peut être obtenu par modifications génétiques (mutation au niveau de gènes de structure d'enzymes impliquées dans la voie de biosynthèse, ou au niveau des séquences permettant l'expression de tels gènes de structure par exemple) ou biochimiques (modification d'un mécanisme post-traductionnel, altération d'un mécanisme de rétroinhibition etc). A cet effet, différents outils de mutagénèse peuvent être utilisés, et notamment:
- des agents physiques : rayons X, rayons Ultra Violet ; ou,
- des agents chimiques tels que :
   . des agents alkylants tels que l'éthylméthane sulfonate (EMS), la N-méthyl-N'-nitro-N-nitrosoguanidine (Delic et al. Mutation Res. 9 (1970) 167-182), ou la 4-nitroquinoléine-1-oxyde (NQO);
   . des agents bialkylants;
   . des agents intercalants ; ou,
- tout système d'insertion mutationnel sur l'ADN, et en particulier les transposons, les plasmides intégratifs, les phages ou les prophages ; ou encore,
- la fusion de protoplastes (Cohen, Nature 268 (1977) 171-174).

Chacun de ces outils peut être appliqué sur les microorganismes non-sélectifs à l'état de spores, de spores germées ou en cours de germination ou sur mycélium. Par ailleurs, ces différents outils peuvent également être utilisés en combinaison.

Bien que certains schémas mutationnels préférés puissent être élaborés conduisant à la formation des microorganismes sélectifs recherchés, il est entendu que l'étape de mutagénèse peut être adaptée par l'homme de l'art en fonction du microorganisme de départ utilisé, de la streptogramine recherchée, et des objectifs fixés (augmentation de la vitesse de synthèse de la composante recherchée, augmentation du niveau de production de celle-ci, réduction de la consommation de matières premières du microorganisme etc). Dans ces conditions, la présente invention n'est pas limitée à un schéma mutationnel précis, mais s'étend à toutes les manipulations (au hasard ou dirigées) permettant d'obtenir des microorganismes capables de produire sélectivement une composante des streptogramines à partir de microorganismes non-sélectifs.

A titre d'exemple spécifique, la mutagénèse en présence d'éthylméthane sulfonate ou par traitement aux UV donne de bons résultats.

En ce qui concerne la seconde étape du procédé, elle permet d'identifier et d'isoler les microorganismes sélectifs. Cette étape peut être réalisée de différentes manières, et notamment au moyen d'un test de sensibilité vis-à-vis d'un germe. A cet effet, différents germes sensibles spécifiquement aux composantes du groupe A ou à celles du groupe B des streptogramines existent. On peut notamment citer Bacillus subtilis (ATCC6633), Bacillus circulans, Bacillus cereus (Watanabe, J. Antibio. Ser. A XIII(1) (1960) 62), ou C.xerosis (Watanabe précitée) qui sont sensibles spécifiquement aux composantes du groupe B. On peut également citer Streptococcus agalactiae B96 (Antimicrob. Agents Chemother. 10(5) (1976) 795), Micrococcus luteus (Prikrylova précitée) ou Sarcina lutea (ATCC9341) qui sont sensibles spécifiquement aux composantes du groupe A. Par ailleurs, Il est également possible de préparer artificiellement des germes sensibles spécifiquement à une composante des streptogramines en insérant, dans un germe sensible aux 2 composantes des streptogramines, un gène de résistance à l'une d'entre-elles. Certains de ces gènes ont en effet été clones (Le Goffic et al., J. Antibio. XXX(8) (1977) 665 ; Le Goffic et al., Ann. Microbiol. Inst. Pasteur 128B (1977) 471 ; Solh et al., Path. Biol. 32(5) (1984) 362), et l'homme du métier peut, en utilisant les techniques classiques de biologie moléculaire, introduire de tels gènes dans différents germes. L'étape de sélection peut également être effectuée par test ELISA au moyen d'anticorps spécifiques des composantes A ou B, ou encore par des techniques analytiques telles que la chromatographie (chromatographie liquide, chromatographie sur couche mince, etc). Dans le cas d'un test de sensibilité vis-à-vis d'un germe, il est en outre préférable de valider la sélection par dosage chromatographique.

Un autre objet de la présente invention concerne un procédé de préparation d'une des composantes A ou B des streptogramines caractérisé en ce que l'on cultive un microorganisme tel que défini ci-avant dans des conditions de production, et on sépare la composante produite du milieu de culture. Les composantes A et B des streptogramines peuvent être séparées du moût de fermentation par les méthodes classiques d'extraction par solvant. Il est notamment possible d'opérer selon le protocole suivant : filtration du moût après acidification à pH 3 environ, neutralisation du filtrat à pH 7, extraction par le dichloréthane, concentration et précipitation de la composante produite par un mauvais solvant tel que l'éther de pétrole. La composante ainsi obtenue peut ensuite être purifiée selon les méthodes connues telles que la chromatographie. Des procédés de purification de streptogramines à partir de moût de fermentation de microorganismes non-sélectifs ont été décrits dans la littérature, et peuvent être appliqués à la présente invention (Cf notamment Chapin et al., J. of Liquid Chromatography 11(11) (1988) 2367 ; Prikrylova et al., Biotechnology and Bioindustry/2 (1988) 20 ; Biot A. Biotechnology of Industrial Antibiotics, vol 22 (1984) p 695).

Dans un mode particulier, l'invention concerne un procédé de préparation d'une macrolactone de formule générale représentée sur la figure 1 dans laquelle les différents substituants sont définis comme précédemment, ou d'un mélange de telles macrolactones, caractérisé en ce que l'on cultive un microorganisme spécifique tel que défini ci-avant, et on sépare les produits obtenus du milieu de culture.

Dans un autre mode particulier, l'invention concerne un procédé de préparation d'un depsipeptide de formule générale représentée sur la figure 2(a) dans laquelle les différents substituants sont définis comme précédemment, ou d'un mélange de tels depsipeptides, caractérisé en ce que l'on cultive un microorganisme spécifique tel que défini ci-avant, et on sépare les produits obtenus du milieu de culture.

L'invention a également pour objet toute composante A d'une Streptogramine quand elle est obtenue à partir du milieu de culture d'un microorganisme producteur sélectif selon l'invention. Préférentiellement, il s'agit d'une macrolactone de formule générale représentée sur la figure 1 dans laquelle les différents substituants sont définis comme précédemment, ou d'un mélange de telles macrolactones.

L'invention a également pour objet toute composante B d'une Streptogramine quand elle est obtenue à partir du milieu de culture d'un microorganisme producteur sélectif selon l'invention. Préférentiellement, il s'agit d'un depsipeptide de formule générale représentée sur la figure 2 dans laquelle les différents substituants sont définis comme précédemment, ou d'un mélange de tels depsipeptides.

Les composantes A et B des streptogramines ainsi obtenues (à partir du milieu de culture de microorganismes sélectifs selon l'invention) peuvent être utilisées pour des études structure-fonction, pour la réalisation de dérivés d'hémisynthèse, ou pour la préparation de compositions antibiotiques.

La présente invention sera plus complètement décrite à l'aide des exemples qui suivent, qui doivent être considérés comme illustratifs et non limitatifs.

### EXEMPLES

### Exemple 1 : Préparation de microorganismes producteurs spécifiques de la pristinamycine PII à partir de la souche DS5647.

Cet exemple a été réalisé sans étape préalable de mutagénèse.

### 1. Sélection par sensibilité à un germe.

Une suspension de spores de Spristinaespiralis DS5647 (ATCC25486) est étalée après dilution sur milieu gélosé complet (milieu HT : dextrine blanche 10 g ; NZ amine type A 2 g ; extrait de levure 1 g ; extrait de viande 1 g ; CoCl₂ 6H₂O 0,02 g ; bacto agar 20 g ; eau déminéralisée q.s.p. 1 l : Cf Pridham, Antibiotic Annual (1956) 957) pour avoir des colonies isolées. Après une étape de croissance, ces colonies isolées sont repiquées sur milieu de production (milieu HT). A un age défini, des carottes sont prélevées sur le milieu afin de doser les pristinamycines produites.

### 1.1. Dosage des pristinamycines PI.

Une partie des carottes prélevées est déposée sur milieu gélosé [25 g/l de milieu Mérieux n° 2 (Mérieux 5 177 1), 20 g/l de NaCl, 5 g/l de tampon TAPS, pH 8,5) additionné par 40 mg/l de pristinamycine PII et ensemencé par 3,6 10⁸ sp/l de Bacillus subtilis ATCC6633. Ce milieu est préparé le jour du carottage de la manière suivante : On fait fondre au micro-ondes le milieu gélosé réparti en erlens de 200 ml, puis la température des culots est ramenée à 50°C environ dans un bain-marie, puis contrôlée sous hotte par un thermomètre plongeant dans le milieu sous agitation légère. La température de 48°C étant atteinte, chaque culot est additionné par :
- 2 ml d'une solution de pristinamycine PII à la concentration de 4 mg/ml (concentration finale 40 mg/l), et,
- 0,4 ml d'une suspension de Bacillus subtilis ATCC6633 à la concentration de 3,6 10⁹sp/ml diluée au vingtième (concentration finale 3,6 10⁸ sp/l).

Le milieu est homogénéisé par agitation douce, puis coulé à raison d'un culot par plaque ("Screening Plate" NUNC) sur une surface rigoureusement plane.

Après dépôt des carottes sur les plaques (diamètre du carotteur : 4,0 mm), une prédiffusion de 6 heures à 4°C est effectuée, puis les plaques sont incubées 16 heures à 37°C.

### 1.2. Dosage des pristinamycines PII.

Une partie des carottes prélevées est déposée, sur milieu gélosé [52 g/l de Brain Heart infusion agar] additionné par 10 mg/l de pristinamycine PI et ensemencé par 5 10⁸ sp/l de Streptococcus agalactiae B96. Ce milieu est préparé le jour du carottage de la manière suivante : On fait fondre au micro-ondes le milieu gélosé réparti en erlens de 200 ml, puis la température des culots est ramenée à 50°C environ dans un bain-marie, puis contrôlée sous hotte par un thermomètre plongeant dans le milieu sous agitation légère. La température de 45°C étant atteinte, chaque culot est additionné par :
- 1 ml d'une solution de pristinamycine PI à la concentration de 2 mg/ml (concentration finale 10 mg/l), et,
- 0,7 ml d'une suspension de Streptococcus agalactiae B96 à la concentration de 5 10⁸ sp/ml.

Le milieu est homogénéisé par agitation douce, puis coulé à raison d'un culot par plaque ("Screening Plate" NUNC) sur une surface rigoureusement plane.

Après dépôt des carottes sur les plaques (diamètre du carotteur : 3.5 mm), une prédiffusion de 6 heures à 4°C est effectuée, puis les plaques sont incubées 16 heures à 37°C.

### 1.3. Résultats

Les diamètres des auréoles d'inhibition sont mesurés pour chacune des plaques contenant les germes test. Ils sont fonction de la concentration d'antibiotique (composante A ou B) présente dans le milieu, et permettent donc d'identifier les clones non producteurs de l'une ou l'autre des composantes.

Sur 3600 clones testés, 36 ont ainsi été identifiés ne produisant pas de pristinamycine PI.

### 2. Sélection par dosage chromatographique.

Les clones ne produisant pas de pristinamycine PI mais produisant de la pristinamycine PII en milieu solide ainsi identifiés sont ensuite testés en milieu liquide. Pour cela, 0,5 ml d'une suspension de spores des souches sélectionnées en 1. sont ajoutés en condition stérile à 40 ml de milieu inoculum dans un erlen de 300 ml. Le milieu inoculum est constitué par 10 g/l de Corn Steep, 15 g/l de saccharose, 10 g/l de (NH₄)₂SO₄, 1 g/l de K₂HPO₄, 3 g/l de NaCl, 0,2 g/l de MgSO₄-7H₂O, et 1,25 g/l de CaCO₃. Le pH est ajusté à 6,9 par de la soude avant l'introduction du carbonate de calcium. Les erlens sont agités pendant 44 heures à 27°C sur un agitateur rotatif à la vitesse de 325 tr/min. 2,5 ml de la culture précédente âgée de 44 heures sont ajoutés stérilement à 30 ml de milieu de production dans un erlen de 300 ml. Le milieu de production est constitué par 25 g/l de farine de soja, 7,5 g/l d'amidon, 22,5 g/l de glucose, 3,5 g/l de levure fourragère, 0,5 g/l de sulfate de zinc et 6 g/l de carbonate de calcium. Le pH est ajusté à 6 avec de l'acide chlorhydrique avant l'ajout du carbonate de calcium. Les erlens sont agités pendant 24 h, 27 h ou 30 h. 10 g de moût sont alors pesés dans un erlen lisse, auxquels sont ajoutés 20 ml de phase mobile composée par 62,5 % d'acétonitrile et 37,5 % d'une solution 0,1 M deK₂HPO₄ concentré. Après agitation sur un agitateur (325 rpm) pendant 20 minutes à température ambiante, le tout est filtré sur filtre de papier et la pristinamycine contenue dans le filtrat est dosée par CPL. Pour cela, 2.4 ml du filtrat sont mis en solution dans un diluant composé d'acétonitrile et d'eau dans un rapport 30/70. Cette solution est injectée sur une colonne SFCC (longueur : 25 cm ; diamètre : 1/4 de pouce ; phase stationnaire : Nucléosil C8 5 microns). La phase mobile est composée de 630 ml de KH₂PO₄ 0,1M (ajusté à pH 3 par H₃PO₄) et de 370 ml d'acétonitrile, et l'élution est effectuée à un débit de 1 ml/minute. Les streptogramines sont détectées par mesure d'absorption à 206 nm par un détecteur LDC Spectromonitor III.

Cette analyse permet de mettre en évidence 3 clones producteurs sélectifs de pristinamycine PII parmi les 36 identifiés précédemment. Le clone Pr4R12 a été déposé le 11 mars 1992 au Centraalbureau voor Schimmelcultures à Baam (Pays-Bas), selon les conditions du Traité de Budapest, sous le numéro CBS 183.92.

### Exemple 2 : Préparation de microorganismes producteurs spécifiques de la pristinamycine PI à partir de la souche DS5647.

Cet exemple comprend une étape de mutagénèse sur le microorganisme non-sélectif, suivie d'une étape de sélection.

### 1. Mutagénèse.

### 1.1. Mutagénèse à l'éthylméthane sulfonate (EMS).

Un premier type de mutagénèse a été réalisé sur la souche DS5647 non-sélective en utilisant comme agent mutagène un agent chimique : l'éthylméthane sulfonate (EMS).

Une suspension de spores de la souche DS5647 est reprise dans 40 ml d'un tampon phosphate 0,2M pH 7 (K₂HPO₄ 0,2M ; pH ajusté à 7 par une solution KH₂PO₄ 0,2M). Cette suspension est ensuite répartie en 4 erlens de 100 ml à raison de 10 ml par erlen, soit environ 6.10⁸ spores/erlen. 0,4 ml d'EMS sont alors ajoutés dans chaque erlen (concentration finale 4 %) et les suspensions sont agitées à 280 rpm à 30°C pendant des temps variables (Cf tableau). Les suspensions sont ensuites transférées dans des gros pots de 250 ml et additionnées de 90 ml d'une solution Na₂S₃O₃, 5H₂O 0,16M. Un échantillon de 0,5 ml est prélevé dans chaque pot et étalé sur boite contenant du milieu HT pour déterminer le taux de survie par comptage par rapport au témoin (Cf tableau). Le reste de la suspension est centrifugé, rincé une fois par 10 ml de Na₂S₃O₃, 5H₂O 0,16M, centrifugé à nouveau, puis repris par 10 ml de milieu Hirsch (J. Bacteriol. 126 (1976) 13). Après une nuit d'expression phénotypique à 30°C sous agitation (280 rpm), les cellules sont étalées sur boîte contenant du milieu HT pour déterminer le taux d'auxotrophie et l'hétérogénéité morphologique des colonies. Pour mesurer le taux d'auxotrophie, les colonies sont repiquées sur milieu minimum et le % de colonies incapables de pousser est déterminé. Le milieu minimum est composé de agar 10 g ; L-asparagine 0,5 g, K₂HPO₄ 0,5 g ; MgSO₄,7H₂O 0,2 g ; FeSO₄,7H₂O 0,01 g ; glucose autoclavé 10 g ; eau distillée 1 l (Hopwood D.A., Bact. Rev. 31 (1967) 373). Les résultats obtenus sont présentés dans le tableau suivant.

| Paramètres | R0 | R1 | R2 | R3 |
|---|---|---|---|---|
| EMS | 0 | 4% | 4% | 4% |
| Temps d'agitation | 150 mn | 130 mn | 150 mn | 180 mn |
| % de survie | 100 | 5,7 | 0,94 | 0,21 |
| % d'auxotrophes | 0,4 | 4,3 | 6,0 | 5,8 |
| Hétérogénéité | +/- | + | +++ | +++ |

### 1.2. Mutagénèse par les UV.

Un deuxième type de mutagénèse a été réalisé sur la souche DS5647 non-sélective en utilisant comme agent mutagène un agent physique : les rayons ultraviolet.

Une suspension de spores de la souche DS5647 (7.10⁸ spores/ml) est diluée avec de l'eau tweenée à 0,01 % (concentration finale : 5.10⁷ spores/ml). Cette suspension est répartie dans des boîtes de pétri à raison de 10 ml par boîte, soit environ 5.10⁸ spores/boîte. Les boîtes sont alors exposées aux UV, à différentes puissances d'irradiation (Cf tableau). Le contenu de chaque boîte est ensuite centrifugé 10 minutes à 3000 tours/minute et les culots sont repris par 5 ml de milieu Hirsch. Les cellules sont maintenues à 30°C sur un agitateur jusqu'à ce que l'on observe un début de germination. Les cellules sont ensuite étalées et les paramètres suivants, témoignant de l'efficacité de la mutagénèse sont déterminés : taux de survie et taux d'auxotrophie. Ces paramètres sont déterminés comme dans l'exemple ci-dessus.

| Paramètres | T0 | T1 | T2 | T3 | T4 | T5 | T6 |
|---|---|---|---|---|---|---|---|
| Puissance d'irradiation ergs/mm2 | 0 | 3420 | 4050 | 5940 | 7200 | 8100 | 9120 |
| Cellules survivantes / ml | 10⁷ | 10⁴ | 10³ | 3.10² | 10² | 4.10² | 10² |
| % de survie | | 0,1 | 0,01 | 0,003 | 0,001 | 0,004 | 0,001 |
| % d'auxotrophes | < 0,4 | 0,4 | 3,2 | 2,8 | 5,6 | 2,8 | 3,8 |

### 2. Sélection des producteurs spécifiques de pristinamycine PI.

La sélection est effectuée comme dans l'exemple 1 en deux étapes (test de sensibilité à un germe en milieu solide, et validation en milieu liquide par analyse chromatographique) à partir des colonies obtenues par mutagénèse EMS (R2). Sur 3600 clones de départ, 6 ont été identifiés en milieu solide, dont 3 ont été confirmés en milieu liquide comme producteurs spécifiques de pristinamycine PI. Le clone Pr4R31 a été déposé le 11 mars 1992 au Centraalbureau voor Schimmelcultures à Baam (Pays-Bas), selon les conditions du Traité de Budapest, sous le numéro CBS 182.92.

### Exemple 3 : Préparation de microorganismes producteurs spécifiques d'ostréogricine A et B à partir de la souche S.ostreogriseus ATCC 27455.

Cet exemple comprend une étape de mutagénèse sur le microorganisme non-sélectif, suivie d'une étape de sélection.

### 1. Mutagénèse.

La mutagénèse a été effectuée au moyen d'éthylméthane sulfonate (EMS).

Une suspension de spores de la souche ATCC 2745 est reprise dans 50 ml d'un tampon phosphate 0,2M pH 7 (K₂HPO₄ 0,2M ; pH ajusté à 7 par une solution KH₂PO₄ 0,2M). Cette suspension est ensuite répartie en 5 erlens de 100 ml à raison de 10 ml par erlen, soit environ 9,3.10⁸spores/erlen. L'EMS est alors ajouté dans chaque erlen à des concentrations variables (cf tableau) et les suspensions sont agitées à 280 rpm à 30°C pendant 150 ou 180 minutes. Les suspensions sont ensuites transférées et traitées comme dans l'exemple 2 point 1.1. Les résultats obtenus sont, présentés dans le tableau suivant.

| Paramètres | QO1 | QO2 | QO3 | QO6 | QO7 |
|---|---|---|---|---|---|
| EMS | 0 | 4% | 4% | 5% | 6% |
| Temps d'agitation | 150 mn | 150 mn | 180 mn | 180 mn | 180 mn |
| % de suivie | 100 | 15,6 | 4,4 | 2,9 | 0,13 |
| % d'auxotrophes | 0 | 2,8 | 3,3 | 2,3 | 4,3 |
| Hétérogénéité | +/- | + | ++ | +++ | +++ |

### 2. Sélection des producteurs spécifiques d'ostréogricine A.

La sélection est effectuée comme dans l'exemple 1 en deux étapes (test de sensibilité à un germe en milieu solide, et validation en milieu liquide par analyse chromatographique) à partir des colonies obtenues par mutagénèse EMS (QO3 et QO6) Sur 1800 clones de chaque traitement mutagène (3600 clones en tout), 4 ont été identifiés en milieu solide (2 pour chaque traitement). 2 souches ont été confirmées en milieu liquide dans les mêmes conditions que dans l'exemple 1, comme producteurs spécifiques d'ostréogricine type A. Le clone Pr4QO63 a été déposé le 27.01.93 au Centraalbureau voor Schimmelcultures à Baarn (Pays-Bas), selon les conditions du Traité de Budapest, sous le numéro CBS 143.93.

### 3. Sélection des producteurs spécifiques d'ostréogricine B.

La sélection est effectuée comme dans l'exemple 1 en deux étapes (test de sensibilité à un germe en milieu solide, et validation en milieu liquide par analyse chromatographique) à partir des colonies obtenues par mutagénèse EMS (QO3). Sur 1800 clones de départ, 1 seul clone a été identifié en milieu solide, qui s'est confirmé en milieu liquide. Ce clone producteur spécifique d'ostréogricine type B, désigné Pr4QO31, a été déposé le 27.01.93 au Centraalbureau voor Schimmelcultures à Baarn (Pays-Bas), selon les conditions du Traité de Budapest, sous le numéro CBS 142.93.

### Exemple 4 : Préparation de microorganismes producteurs spécifiques de virginiamycine A et B à partir de la souche S.virginiae ATCC 13161.

Cet exemple comprend une étape de mutagénèse sur le microorganisme non-sélectif, suivie d'une étape de sélection.

### 1. Mutagénèse.

La mutagénèse a été effectuée au moyen d'éthylméthane sulfonate (EMS), comme dans l'exemple 3, sur une suspension de spores répartie en 5 erlens de 100 ml à raison de 10 ml par erlen, soit environ 3,2.10⁹ spores/erlen. Les conditions de la mutagénèse et les résultats obtenus sont rassemblés dans le tableau suivant. Le protocole décrit dans l'exemple 3 a été suivi.

| Paramètres | QV4 | QV5 | QV6 | QV9 | QV7 | QV10 |
|---|---|---|---|---|---|---|
| EMS | 0 | 0,5 % | 1 % | 1,5 % | 2 % | 3 % |
| Temps d'agitation | 100 mn | 100 mn | 100 mn | 100 mn | 100 mn | 100 mn |
| % de survie | 100 | 72 | 55 | 25 | 10 | 0,7 |
| % d'auxotrophes | < 0,4 | 0,7 | 8,3 | 4,6 | 4,2 | 3,1 |
| Hétérogénéité | +/- | +/- | +/- | ++ | + | ++ |

### 2. Sélection des producteurs spécifiques de virginiamycine VM.

La sélection est effectuée comme dans l'exemple 1 en deux étapes (test de sensibilité à un germe en milieu solide, et validation en milieu liquide par analyse chromatographique) à partir des colonies obtenues par mutagénèse EMS (QV9 et QV7). Sur 1800 clones de chaque traitement mutagène (3600 clones en tout), 2 ont été identifiés en milieu solide, selon le protocole décrit dans l'exemple 1 point 1.2 (en utilisant 10⁷ sp/l de Streptococcus B96). Ces 2 souches ont été confirmées en milieu liquide dans les conditions de l'exemple 1, comme producteurs spécifiques de virginiamycine type VM. Le clone Pr4QV71 a été déposé le 27.01.93 au Centraalbureau voor Schimmelcultures à Baarn (Pays-Bas), selon les conditions du Traité de Budapest, sous le numéro CBS 140.93.

Pour la sélection en milieu liquide toutefois, le protocole de l'exemple 1 point 2 a été suivi en utilisant, comme milieu inoculum et comme milieu de production les milieux suivants :
Milieu inoculum : Corn steep atomisé, 20g; farine de soja 10g; autolysat de levure, 5g; huile d'arachide 15g; glucode anhydre 40g; MnSO4 0,01g; CaCO3 5g; eau qsp 11. Le pH est ajusté à 6,80 par de la soude avant l'introduction du carbonate de calcium.
Milieu de production : Corn steep atomisé, 20g; autolysat de levure, 5g; huile d'arachide 10g; glucode anhydre 5g; glycérol 25g; huile de lin 10g; CaCO3 5g; eau qsp 11. Le pH est ajusté à 6,80 par de la soude avant l'introduction du carbonate de calcium.

### 3. Sélection des producteurs spécifiques de virginiamycine type VS.

La sélection est effectuée comme dans l'exemple 1 en deux étapes (test de sensibilité à un germe en milieu solide, et validation en milieu liquide par analyse chromatographique) à partir des colonies obtenues par mutagénèse EMS (QV9). Sur 3700 clones de départ, 1 seul clone a été identifié en milieu solide (Cf protocole exemple 1 point 1.1), qui s'est confirmé en milieu liquide (Cf ci-dessus pour les milieux). Ce clone producteur spécifique de virginiamycine type VS, désigné Pr4QV91, a été déposé le 27.01.93 au Centraalbureau voor Schimmelcultures à Baarn (Pays-Bas), selon les conditions du Traité de Budapest, sous le numéro CBS 141.93.

**TABLEAU 1**

| **MICROORGANISMES** | **ANTIBIOTIQUES** |
|---|---|
| **CHAMPIGNONS** | |
| | |
| Micromonospora sp. | vernamycine |
| | |
| **STREPTONFYCES** | |
| | |
| S.alborectus | virginiamycine |
| S.conganensis (ATCC13528) | F1370A,B |
| S.diastaticus | plauracine ,streptogramine |
| S.graminofaciens | streptogramine |
| S.griseus (NRRL2426) | viridogriséine |
| S.griséoviridus | griséoviridine |
| S.griséoviridus (FERM P3562) | néoviridogriséine |
| S.lavendulae | étamycine |
| S.loïdensis (ATCC11415) | vernamycine |
| S.mitakaensis (ATCC15297) | mikamycine |
| S.olivaceus (ATCC12019) | synergistine |
| S.ostréogriséus (ATCC27455) | ostréogrycine |
| S.pristinaespiralis (ATCC25486) | pristinamycine |
| S.virginiae (ATCC13161) | virginiamycine |
| S.antibioticus H-827 | yakusimycine |
| S.kurssanovii (ATCC15824) | A14725 |
| S.komoroensis 1753-Z3 | 1745-Z3 |
| Nocardiopsis flava (ATCC29533) | madumycine |
| | |
| **ACTINOMYCES** | |
| | |
| A.auranticolor (ATCC31011) | plauracine |
| A.azureus (ATCC31157) | plauracine |
| A.daghestanicus | etamycine |
| Actinoplanes sp. | A17002 |
| Actinoplanes sp. (ATCC33002) | A15104 |

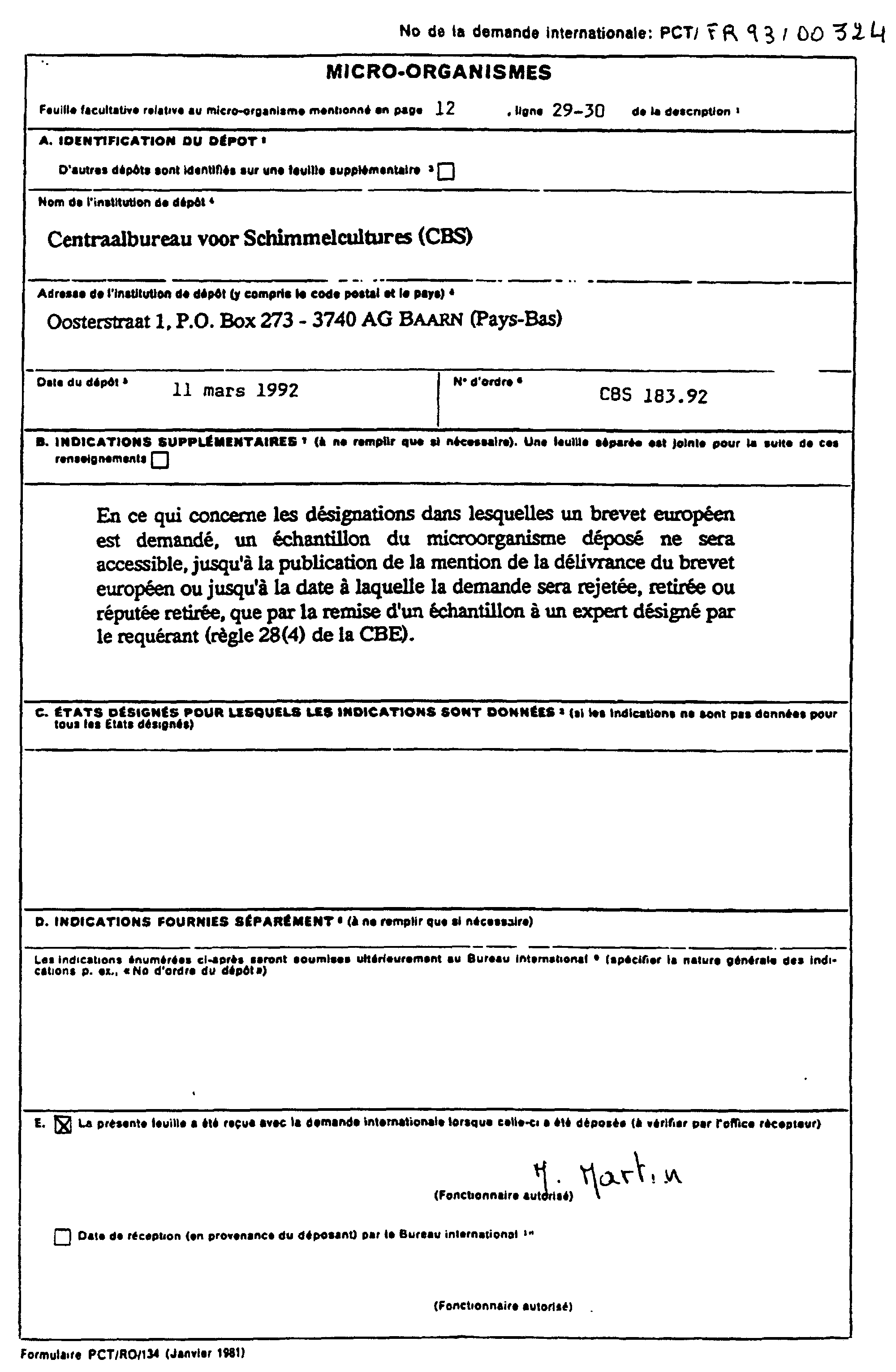

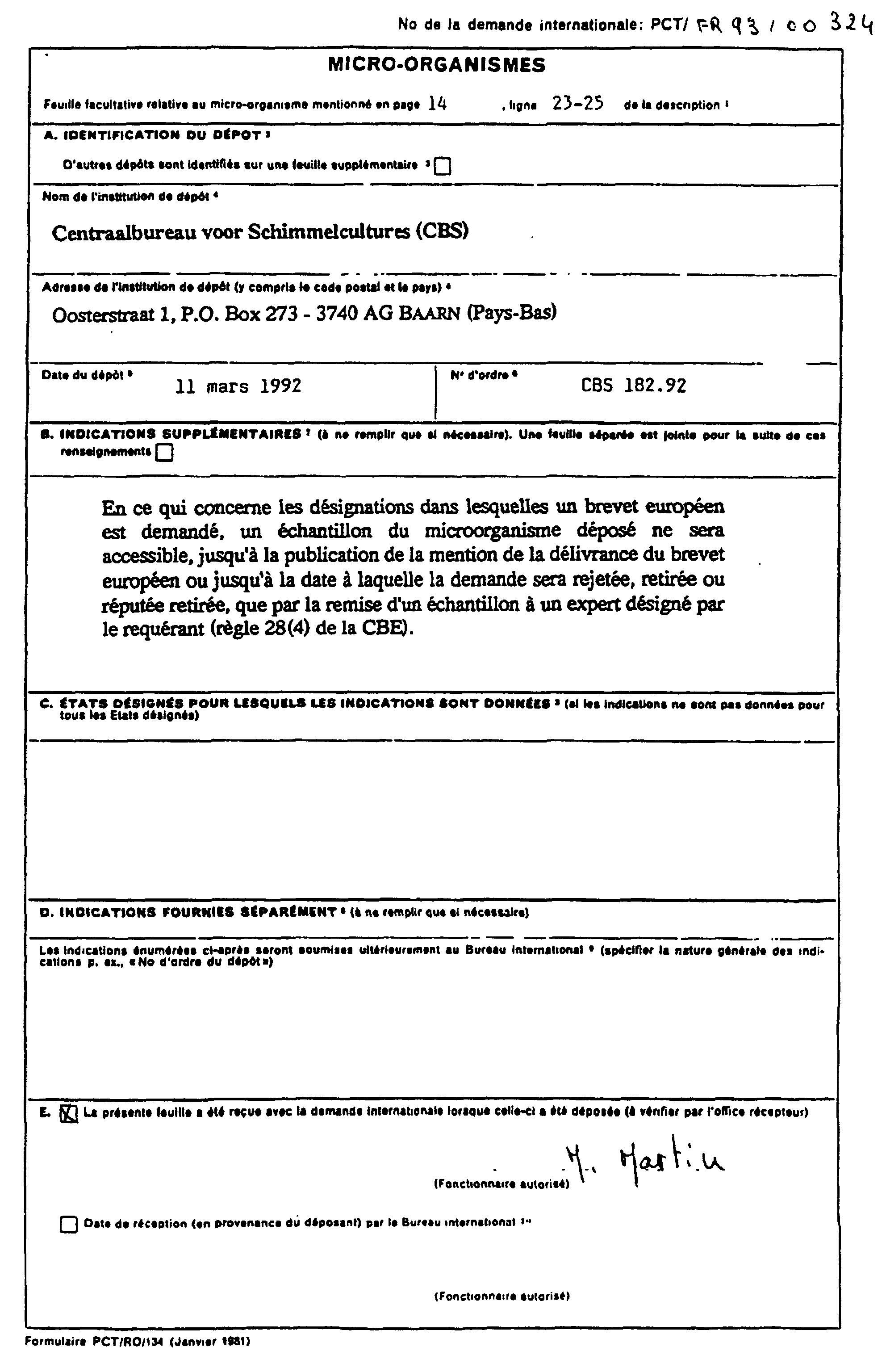

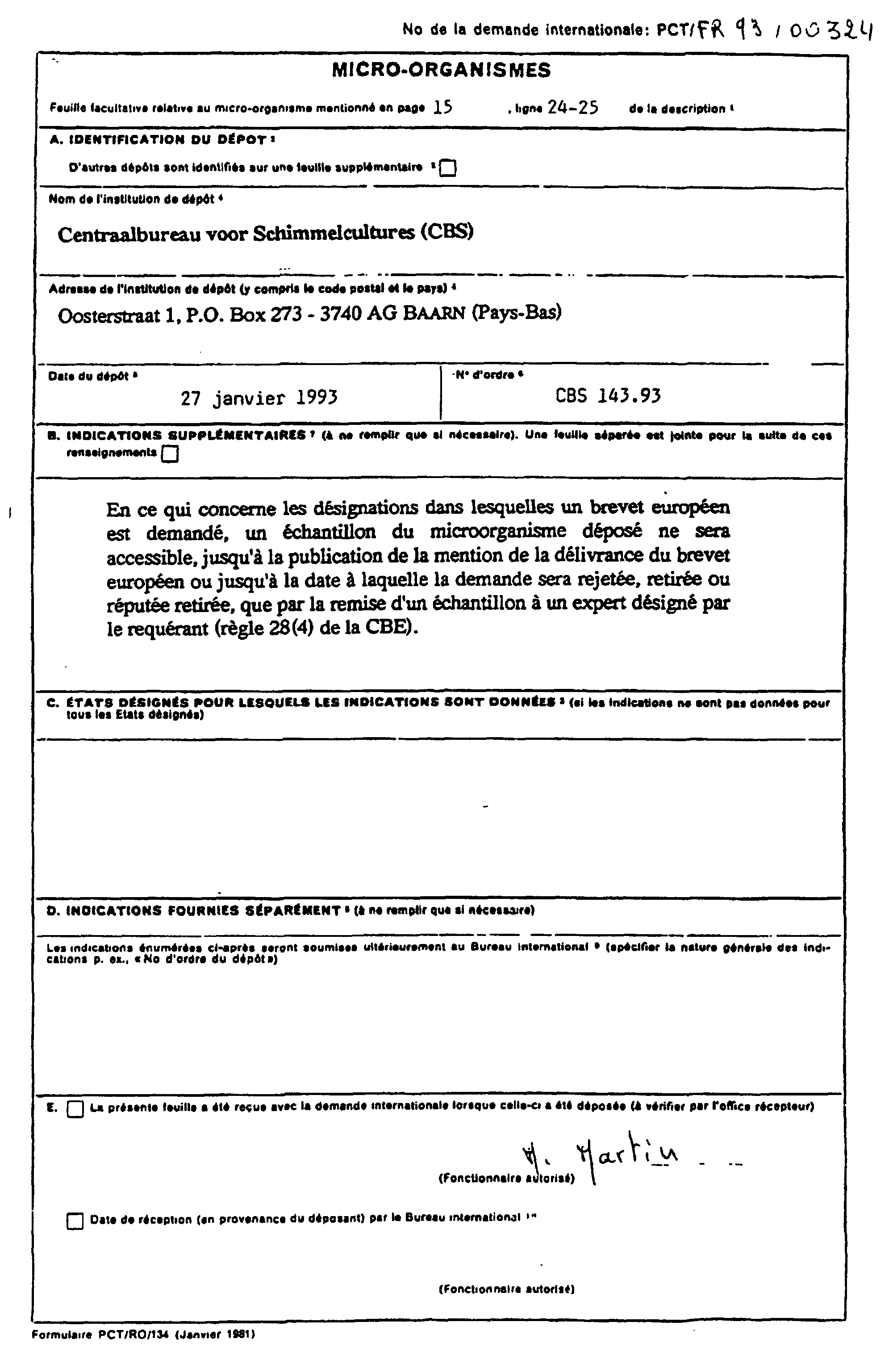

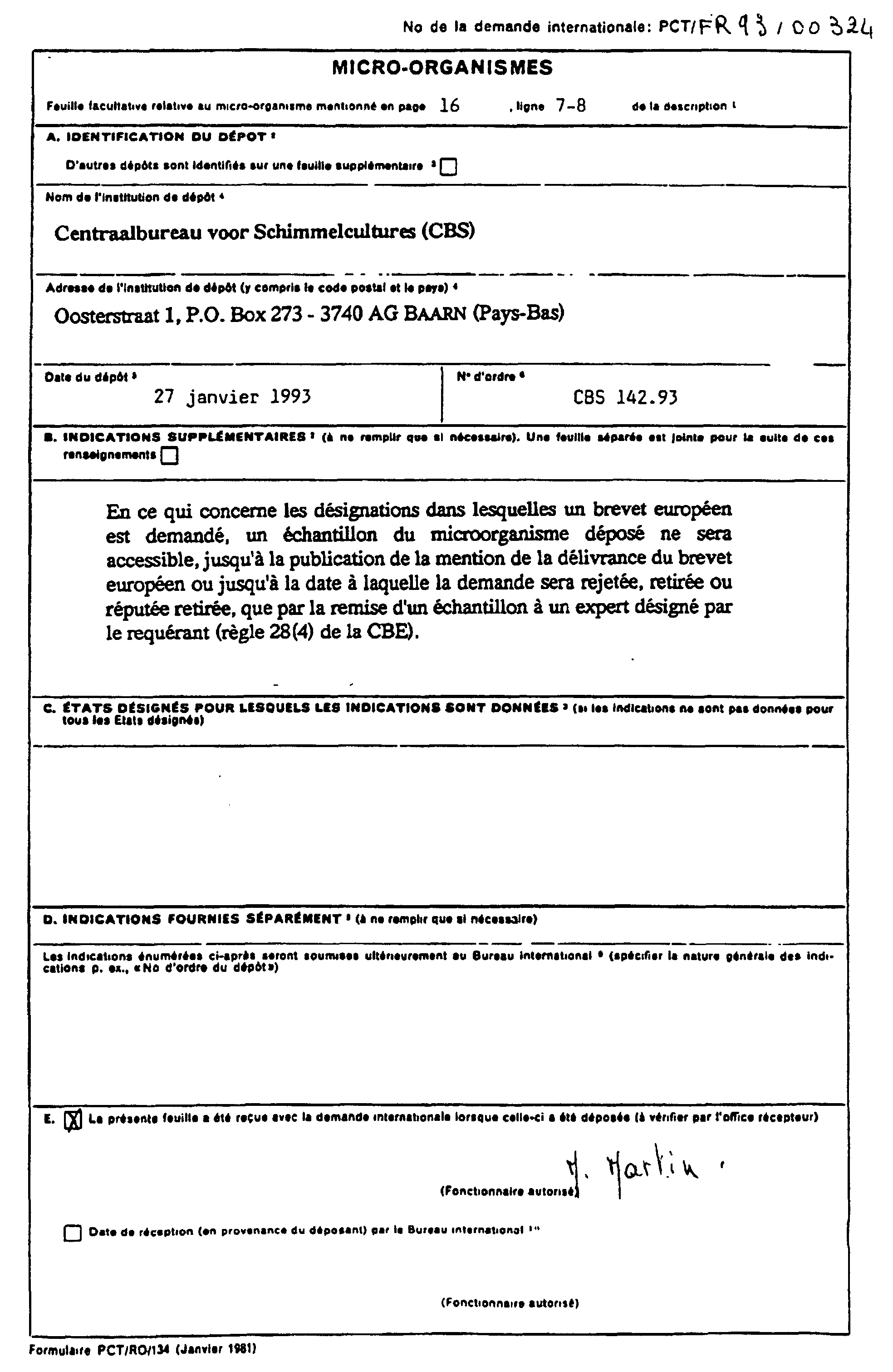

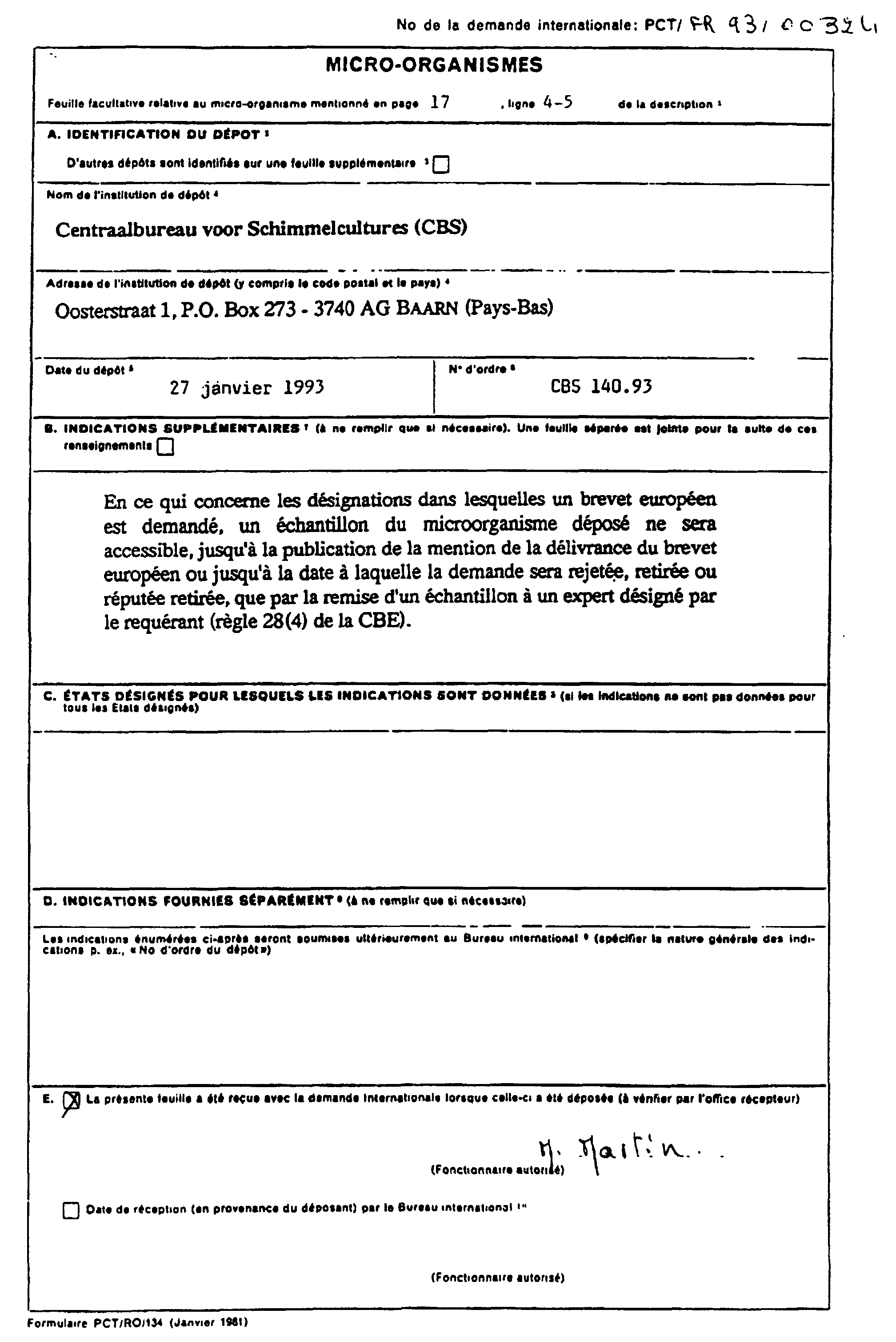

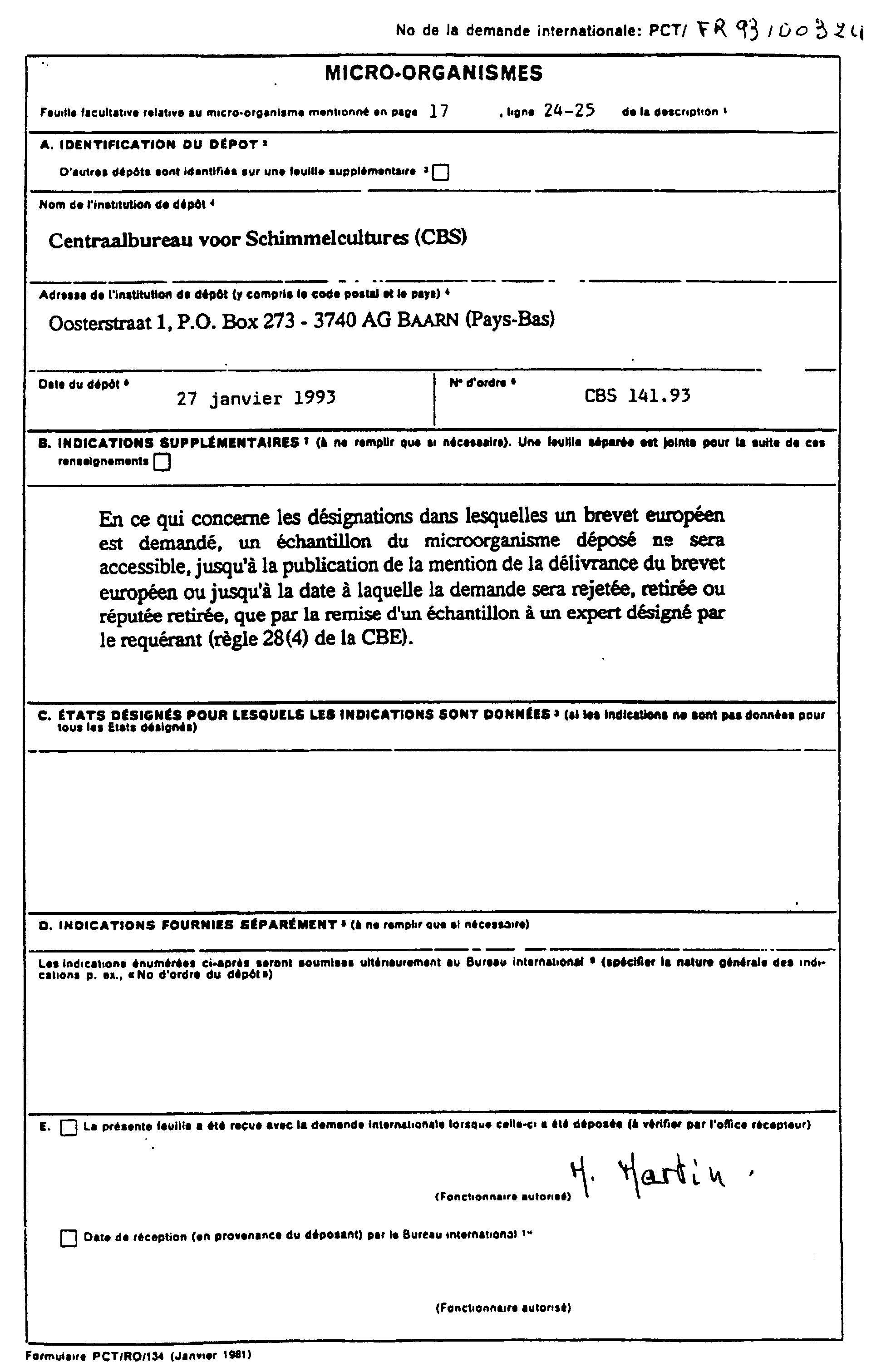

## Revendications

1. Microorganisme capable de produire sélectivement les composantes A ou B des streptogramines.

2. Microorganisme selon la revendication 1 **caractérisé en ce qu'**il produit sélectivement la composante A des streptogramines.

3. Microorganisme selon la revendication 1 **caractérisé en ce qu'**il produit sélectivement la composante B des streptogramines.

4. Microorganisme selon l'une des revendications 1 à 3 **caractérisé en ce que** la streptogramine est choisie parmi le groupe comprenant la pristinamycine, la virginiamycine, la mikamycine, l'ostréogrycine, la synergistine, la viridogriséine, la vernamycine, la plauracine, l'étamycine, la griséoviridine, la néoviridogriséine et la streptogramine.

5. Microorganisme selon la revendication 4 **caractérisé en ce qu'**il produit sélectivement la composante A des streptogramine correspondant à la formule générale présentée sur la figure 1 dans laquelle Y représente une D-proline, une 4,5-déhydroproline, une D-alanine ou une D-cystéine, R0 représente un groupement C=O ou CHOH, R1 est un atome d'hydrogène ou un groupement méthyl, R2 est un atome d'hydrogène ou un groupement méthyl et R3 est un groupement méthyl ou isopropyl.

6. Microorganisme selon la revendication 4 **caractérisé en ce qu'**il produit sélectivement la composante B des streptogramine correspondant à la formule générale présentée sur la figure 2(a) dans laquelle Z représente une L-proline, un acide aspartique L, un acide pipécolique, un acide 4-oxopipécolique, un acide 4-hydroxy-pipécolique L ou un acide 5-hydroxy-4-oxopipécolique L, R4 est un atome d'hydrogène ou une amine de formule NH(CH3) ou N(CH3)2, R5 est un atome d'hydrogène ou un groupement méthyl et R6 est un groupement méthyl ou éthyl.

7. Microorganisme selon la revendication 4 **caractérisé en ce qu'**il produit sélectivement la composante B des streptogramines correspondant à la formule générale présentée sur la figure 2(b) dans laquelle R7 représente un atome d'hydrogène ou un groupe hydroxy et R8 représente un groupement méthyl ou éthyl.

8. Microorganisme selon la revendication 4 **caractérisé en ce que** la streptogramine est choisie parmi le groupe comprenant la pristinamycine, la virginiamycine, l'ostréogrycine et la mikamycine.

9. Microorganisine-selon l'une quelconque des revendications 1 à 8 **caractérisé en ce qu'**il s'agit d'un Actinomycète ou d'un champignon.

10. Microorganisme selon la revendication 9 **caractérisé en ce qu'**il s'agit d'un Streptomyces.

11. Les souches S.pristinaespiralis n° CBS 182.92 et CBS 183.92; S.ostreogriseus n° CBS 142.93 et 143.93; et S.virginiae n° CBS 140.93 et 141.93.

12. Procédé de préparation d'un microorganisme tel que défini dans l'une des revendications précédentes **caractérisé en ce que** l'on réalise les étapes suivantes:
- dans une première étape facultative, on effectue une mutagénèse sur un microorganisme producteur de streptogramines non-sélectif, et,
- dans une deuxième étape, on sélectionne les microorganismes sélectifs.

13. Procédé selon la revendication 12 **caractérisé en ce que** le microorganisme producteur de streptogramines non-sélectif est choisi parmi les actinomycètes et les champignons.

14. Procédé selon la revendication 13 **caractérisé en ce que** le microorganisme est un microorganisme producteur non-sélectif d'une streptogramine choisie parmi le groupe comprenant la pristinamycine, la virginiamycine, la mikamycine, l'ostréogrycine, la synergistine, la viridogriséine, la vernamycine, la plauracine, l'étamycine, la griséoviridine, la néoviridogriséine et la streptogramine.

15. Procédé selon la revendication 14 **caractérisé en ce que** le microorganisme est choisi parmi Streptomyces alborectus, Streptomyces diastaticus, Streptomyces graminofaciens, Streptomyces grisous, Streptomyces loïdensis, Streptomyces mitakaensis, Streptomyces olivaceus, Streptomyces ostréogriseus, Streptomyces pristinaespiralis, Streptomyces virginiae, Streptomyces lavendulae, Streptomyces griséoviridus, Micromonospora sp., Actinomyces auranticolor, Actinomyces daghestanicus et Actinomyces azureus.

16. Procédé selon l'une des revendications 12 à 15 **caractérisé en ce que** l'étape de mutagénèse est réalisée par traitement chimique et/ou physique et/ou génétique du microorganisme.

17. Procédé selon l'une des revendications 12 à 15 **caractérisé en ce que** l'étape de sélection est réalisée par un test de sensibilité vis-à-vis d'un germe, ou par un test ELISA au moyen d'anticorps spécifiques des composantes A ou B, ou encore par des techniques analytiques telles que la chromatographie.

18. Procédé selon la revendication 17 **caractérisé en ce que** l'étape de sélection est réalisée par un test de sensibilité vis-à-vis d'un germe suivi d'un dosage chromatographique.

19. Procédé de préparation d'une des composantes A ou B des streptogramines **caractérisé en ce que** l'on cultive un microorganisme selon l'une quelconque des revendications 1 à 11 dans des conditions de production, et on récupère la composante produite.

20. Procédé selon la revendication 19 pour la préparation d'une macrolactone de formule générale représentée sur la figure 1 dans laquelle les substituants ont la même signification que dans la revendication 5, ou d'un mélange de telles macrolactones, **caractérisé en ce que** l'on cultive un microorganisme spécifique selon la revendication 2, et on sépare les produits obtenus du milieu de cultue.

21. Procédé selon la revendication 19 pour la préparation d'un depsipeptide de formule générale représentée sur la figure 2(a) dans laquelle les substituants ont la même signification que dans la revendication 6, ou d'un mélange de tels depsipeptides, **caractérisé en ce que** l'on cultive un microorganisme spécifique selon la revendication 3, et on sépare les produits obtenus du milieu de cultue.

22. Utilisation des composantes A et B des streptogramines obtenues selon le procédé de l'une des revendications 12 à 21 pour la réalisation de dérivés d'hémisynthèse.

## Patentansprüche

1. Mikroorganismus, der fähig ist, die Komponente A oder B der Streptogramine zu produzieren.

2. Mikroorganismus nach Anspruch 1, **dadurch gekennzeichnet, daß** er selektiv die Komponente A der Streptogramine produziert.

3. Mikroorganismus nach Anspruch 1, **dadurch gekennzeichnet, daß** er selektiv die Komponente B der Streptogramine produziert.

4. Mikroorganismus nach einem der Ansprüche 1 bis 3, **dadurch gekennzeichnet, daß** das Streptogramin aus der Gruppe ausgewählt ist, die Pristinamycin, Virginiamycin, Mikamycin, Ostreogrycin, Synergistin, Viridogrisein, Vernamycin, Plauracin, Etamycin, Griseoviridin, Neoviridogrisein und Streptogramin umfaßt.

5. Mikroorganismus nach Anspruch 4, **dadurch gekennzeichnet, daß** er selektiv die Komponente A der Streptogramine produziert, die der allgemeinen Formel entspricht, die in Figur 1 dargestellt ist, worin Y ein D-Prolin, ein 4,5-Dehydroprolin, ein D-Alanin oder ein D-Cystein darstellt, RO eine Gruppierung C=O oder CHOH darstellt, R1 ein Wasserstoffatom oder eine Methyl-Gruppierung ist und R3 eine Methyl- oder Isopropyl-Gruppierung ist.

6. Mikroorganismus nach Anspruch 4, **dadurch gekennzeichnet, daß** er selektiv die Komponente B der Streptogramine produziert, die der allgemeinen Formel entspricht, die in Figur 2(a) dargestellt ist, worin Z ein L-Prolin, eine L-Asparaginsäure, eine Pipecolinsäure, eine 4-Oxopipecolinsäure, eine L-4-Hydroxypipecolinsäure oder eine L-5-Hydroxy-4-oxopipecolinsäure darstellt, R₄ ein Wasserstoffatom oder ein Amin der Formel (NH(CH3) oder N(CH3)2 ist, R5 ein Wasserstoffatom oder ein Methyl-Gruppierung ist und R6 eine Methyl- oder Ethyl-Gruppierung ist.

7. Mikroorganismus nach Anspruch 4, **dadurch gekennzeichnet, daß** er selektiv die Komponente B der Streptogramine produziert, die der allgemeinen Formel entspricht, die in Figur 2(b) dargestellt ist, worin R7 ein Wasserstoffatom oder ein Hydroxy-Gruppe darstellt und R8 eine Methyl- oder Ethyl-Gruppierung darstellt.

8. Mikroorganismus nach Anspruch 4, **dadurch gekennzeichnet, daß** das Streptogramin aus der Gruppe, die Pristinamycin, Virginiamycin, Ostreogrycin und Mikamycin umfaßt, ausgewählt ist.

9. Mikroorganismus nach einem der Ansprüche 1 bis 8, **dadurch gekennzeichnet, daß** es sich um einen Actinomyceten oder einen Champignon handelt.

10. Mikroorganismus nach Anspruch 9, **dadurch gekennzeichnet, daß** es sich um einen Streptomyceten handelt.

11. Die Stämme S. pritinaespiralis, Nr. CBS 182.92 und CBS 183.92; S. ostreogriseus, Nr. CBS 142.93 und 143.93, und S. virginiae, Nr. CBS 140.93 und 141.93.

12. Verfahren zur Herstellung eines Mikroorganismus, wie er in einem der vorangehenden Ansprüche definiert ist, **dadurch gekennzeichnet, daß** man die folgenden Stufen durchführt:
- in einer ersten fakultativen Stufe führt man eine Mutagenese an einem Mikroorganismus durch, der nicht-selektiver Produzent von Streptograminen ist, und
- in einer zweiten Stufe selektiert man die selektiven Mikroorganismen.

13. Verfahren nach Anspruch 12, **dadurch gekennzeichnet, daß** der Mikroorganismus, der nicht-selektiver Produzent von Streptograminen ist, unter den Actinomyceten und den Champignons ausgewählt wird.

14. Verfahren nach Anspruch 13, **dadurch gekennzeichnet, daß** der Mikroorganismus ein Mikroorganismus ist, der ein nicht-selektiver Produzent eines Streptogramins, ausgewählt aus der Gruppe umfassend Pristinamycin, Virginiamycin, Mikamycin, Ostreogrycin, Synergistin, Viridogrisein, Vernamycin, Plauracin, Etamycin, Griseoviridin, Neoviridogrisein und Streptogramin ist.

15. Verfahren nach Anspruch 14, **dadurch gekennzeichnet, daß** der Mikroorganismus aus Streptomyces alborectus, Streptomyces diastaticus, Streptomyces graminofaciens, Streptomyces griseus, Streptomyces loidensis, Streptomyces mitakaensis, Streptomyces olivaceus, Streptomyces ostreogriseus, Streptomyces pristinaespiralis, Streptomyces virginiae, Streptomyces lavendulae, Streptomyces griseoviridus, Micromonospora sp., Actinomyces auranticolor, Actinomyces daghestanicus und Actonmyces azureus ausgewählt wird.

16. Verfahren nach einem der Ansprüche 12 bis 15, **dadurch gekennzeichnet, daß** die Stufe der Mutagenese durch chemische und/oder physikalische und/oder genetische Behandlung des Mikroorganismus durchgeführt wird.

17. Verfahren nach einem der Ansprüche 12 bis 15, **dadurch gekennzeichnet, daß** die Stufe der Selektion durch einen Test auf Empfindlichkeit gegenüber einem Keim oder durch einen ELISA-Test mittels spezifischer Antikörper für den Bestandteil A oder B oder auch durch analytische Techniken wie Chromatographie durchgeführt wird.

18. Verfahren nach Anspruch 17, **dadurch gekennzeichnet, daß** die Stufe der Selektion durch einen Test auf Empfindlichkeit gegenüber einem Keim im Anschluß an eine chromatographische Analyse durchgeführt wird.

19. Verfahren zur Herstellung von einer der Bestandteile A oder B der Streptogramine, **dadurch gekennzeichnet, daß** man einen Mikroorganismus nach einem der Ansprüche 1 bis 11 unter Produktionsbedingungen kultiviert und den produzierten Bestandteil gewinnt.

20. Verfahren nach Anspruch 19 zur Herstellung eine Makrolactons der allgemeinen Formel, die in Figur 1 dargestellt ist, worin die Substituenten dieselbe Bedeutung wie in Anspruch 5 haben oder eines Gemisches solcher Makrolactone, **dadurch gekennzeichnet, daß** man einen spezifischen Mikroorganismus nach Anspruch 2 kultiviert und die erhaltenen Produkte vom Kulturmedium abtrennt.

21. Verfahren nach Anspruch 19 zur Herstellung eines Depsipeptids der allgemeinen Formel, die in Figur 2(a) dargestellt ist, worin die Substituenten dieselbe Bedeutung wie in Anspruch 6 haben, oder eines Gemisches derartiger Depsipeptide, **dadurch gekennzeichnet, daß** man einen spezifischen Mikroorganismus nach Anspruch 3 kultiviert und die erhaltenen Produkte vom Kulturmedium abtrennt.

22. Verwendung der Komponenten A und B der Streptogramine, erhalten nach dem Verfahren nach einem der Ansprüche 12 bis 21 zur Herstellung von Derivaten der Hemisynthese.

## Claims

1. Microorganism capable of selectively producing the streptogramin A or B components.

2. Microorganism according to Claim 1, **characterized in that** it selectively produces the streptogramin A component.

3. Microorganism according to Claim 1, **characterized in that** it selectively produces the streptogramin B component.

4. Microorganism according to one of Claims 1 to 3, **characterized in that** the streptogramin is chosen from the group consisting of pristinamycin, virginiamycin, mikamycin, ostreogrycin, synergistin, viridogrisein, vernamycin, plauracin, etamycin, griseoviridin, neoviridogrisein and streptogramin.

5. Microorganism according to Claim 4, **characterized in that** it selectively produces the streptrogramin A component corresponding to the general formula shown in Figure 1, in which Y represents a D-proline, a 4,5-dehydroproline, a D-alanine or a D-cysteine, R0 represents a group C=O or CHOH, R1 is a hydrogen atom or a methyl group, R2 is a hydrogen atom or a methyl group and R3 is a methyl or isopropyl group.

6. Microorganism according to Claim 4, **characterized in that** it selectively produces the streptogramin B component corresponding to the general formula shown in Figure 2(a), in which Z represents an L-proline, an L-aspartic acid, a pipecolic acid, a 4-oxopipecolic acid, a 4-hydroxy-L-pipecolic acid or a 5-hydroxy-4-oxo-L-pipecolic acid, R4 is a hydrogen atom or an amine of formula NH(CH₃) or N(CH₃)₂, R5 is a hydrogen atom or a methyl group and R6 is a methyl or ethyl group.

7. Microorganism according to Claim 4, **characterized in that** it selectively produces the streptogramin B component corresponding to the general formula shown in Figure 2(b), in which R7 represents a hydrogen atom or a hydroxyl group and R8 represents a methyl or ethyl group.

8. Microorganism according to Claim 4, **characterized in that** the streptogramin is chosen from the group consisting of pristinamycin, virginiamycin, ostreogrycin and mikamycin.

9. Microorganism according to any one of Claims 1 to 8, **characterized in that** it is an Actinomycete or a fungus.

10. Microorganism according to Claim 9, **characterized in that** it is a Streptomyces.

11. The strains S. pristinaespiralis No. CBS 182.92 and CBS 183.92; S. ostreogriseus No. CBS 142.93 and 143.93; and S. virginiae No. CBS 140.93 and 141.93.

12. Method for preparing a microorganism as defined in any one of the preceding claims, **characterized in that** the following steps are carried out:
- in an optional first step, mutagenesis is carried out on a non-selective streptogramin-producing microorganism, and
- in a second step, the selective microorganisms are selected.

13. Method according to Claim 12, **characterized in that** the non-selective streptogramin-producing microorganism is chosen from actinomycetes and fungi.

14. Method according to Claim 13, **characterized in that** the microorganism is a microorganism which is a non-selective producer of a streptogramin chosen from the group consisting of pristinamycin, virginiamycin, mikamycin, ostreogrycin, synergistin, viridogrisein, vernamycin, plauracin, etamycin, griseoviridin, neoviridogrisein and streptogramin.

15. Method according to Claim 14, **characterized in that** the microorganism is chosen from Streptomyces alborectus, Streptomyces diastaticus, Streptomyces graminofaciens, Streptomyces griseus, Streptomyces loidensis, Streptomyces mitakaensis, Streptomyces olivaceus, Streptomyces ostreogriseus, Streptomyces pristinaespiralis, Streptomyces virginiae, Streptomyces lavendulae, Streptomyces griseoviridus, Micromonospora sp., Actinomyces auranticolor, Actinomyces daghestanicus and Actinomyces azureus.

16. Method according to one of Claims 12 to 15, **characterized in that** the mutagenesis step is carried out by chemical and/or physical and/or genetic treatment of the microorganism.

17. Method according to one of Claims 12 to 15, **characterized in that** the selection step is carried out using a test for sensitivity with respect to a microorganism, using an ELISA assay by means of antibodies specific for the A or B components, or else using analytical techniques such as chromatography.

18. Method according to Claim 17, **characterized in that** the selection step is carried out using a test for sensitivity with respect to a microorganism, followed by chromatographic assaying.

19. Method for preparing one of the streptogramin A or B components, **characterized in that** a microorganism according to any one of Claims 1 to 11 is cultured under production conditions, and the component produced is recovered.

20. Method according to Claim 19, for preparing a macrolactone of general formula represented in Figure 1, in which the substituents have the same meaning as in Claim 5, or a mixture of such macrolactones, **characterized in that** a specific microorganism according to Claim 2 is cultured, and the products obtained are separated from the culture medium.

21. Method according to Claim 19, for preparing a depsipeptide of general formula represented in Figure 2(a), in which the substituents have the same meaning as in Claim 6, or a mixture of such depsipeptides, **characterized in that** a specific microorganism according to Claim 3 is cultured, and the products obtained are separated from the culture medium.

22. Use of the streptogramin A and B components obtained according to the method of one of Claims 12 to 21, for producing hemisynthetic derivatives.
